# EUROPEAN PATENT SPECIFICATION

(11) **EP 1 011 482 B1**
(45) Date of publication and mention of the grant of the patent: **06.12.2006**
(21) Application number: 97944370.2
(22) Date of filing: 25.09.1997
(51) Int. Cl.: A61B 17/22

(54) **AN IN-VITRO METHOD AND A DEVICE FOR ELECTRO MICROSURGERY IN A PHYSIOLOGICAL LIQUID ENVIRONMENT**
IN-VITRO METHODE UND VORRICHTUNG FÜR ELEKTRO-MIKROCHIRURGIE IN EINER UMGEBUNG AUS EINER PHYSIOLOGISCHEN FLÜSSIGKEIT
PROCEDE IN-VITRO ET DISPOSITIF POUR ELECTROMICROCHIRURGIE DANS UN ENVIRONNEMENT COMPOSE DE LIQUIDE PHYSIOLOGIQUE

(30) Priority: 26.09.1996 IL 11930596
(43) Date of publication of application: 28.06.2000
(73) Proprietor: Lewis, Aaron, Jerusalem 93707 (IL)
(72) Inventor: LEWIS, Aaron, Jerusalem (IL); PALANKER, Daniel, Jerusalem (IL); TUROVETS, Igor, Jerusalem (IL)
(74) Representative: Dreiss, Fuhlendorf, Steimle & Becker
(86) International application number: PCT/US1997/016927
(87) International publication number: WO 1998/012974

(56) References cited:
- US-A- 4 476 862
- US-A- 4 597 388
- US-A- 4 674 499
- US-A- 4 901 709
- US-A- 5 254 121

## Description

### Field of the Invention

The invention is a device and a method of using it for cutting in-vitro cell aggregates that allows for the electrical emulation of the microsurgical abilities of pulsed lasers. These lasers are the basis for new laser procedures that are responsible for high speed and high precision cutting action in biological liquid environments with minimal damage to the surrounding mechanism. They work by producing cavitation bubbles. The action of these lasers and laser procedures are simulated in the current invention by using protocols that are a part of this invention. These protocols regulate the electrical current injected into the specialized device that has been devised. As a result it is possible to emulate the cavitation bubble formation cutting that has become a hallmark of various pulsed laser procedures. The invention allows the same device to be used for such an emulation of these cavitation bubbles while also allowing for heat induced coagulation and poration of biological materials.

### Background of the Invention

Various pulsed lasers have been applied to soft tissue cutting and removal in a liquid environment. Three basically different mechanisms of light absorption have- been involved in laser surgery: (1) linear absorption of light by tissue (I. Turovets, D. Palanker, Y. Kokotov, I. Hemo, A. Lewis, *J. Appl. Phys. **79(5):*** 2689-2693 (1996); D. Palanker, I. Turovets, A. Lewis, *Laser-Tissue Interaction VII, Proc. SPIE* **2681** (1996)); (2) linear absorption of light by the medium (P. D. Brazitikos, D. J. D'Amico, M. T. Bernal, A. W. Walsh, *Ophthalmology* **102(2):** 278-290 (1994); C. P. Lin, D. Stern, C. A. Puliafito, *Invest. Ophthalmol. Vis. Sci* **31(12):** 2546-2550 (1990)); or (3) non-linear absorption by tissue or medium associated with dielectric breakdown of the material (A. Vogel, S. Busch, K. Jungnickel, R. Birngruber, *Lasers Surg. Med.* **15**: 32-43 (1994)). In spite of differences in the mechanisms of laser radiation absorption in tissue, the mechanism of tissue disruption with pulsed lasers has been generally associated with an explosive expansion of overheated liquid and subsequent cavitation bubble formation. As a result, material disruption occurs in the light absorption zone and in a zone of fast expansion and collapse of cavitation bubbles (I. Turovets, D. Palanker, Y. Kokotov, I. Hemo, A. Lewis, *J. Appln. Phys. **79(5)**:* 2689-2693 (1996); P. D. Brazitijos, D. J. D'Amico, M. T. Bernal, A. W. Walsh, *Ophthalmology* **102(2)**: 278-290 (1994); and A. Vogel, S. Busch, K. Jungnickel, R. Birngruber, *Lasers Surg. Med.* **15**: 32-43 (1994)) .

In view of the general complicated nature of laser-based devices a search was made for ways to emulate with non-laser methodologies the mechanisms that are known to occur with available lasers. In the case of cavitation bubble generation it is known, as described above, that these bubbles result from local and fast heat energy deposition. Thus, it is logical to consider an overheating of a conductive medium with a short pulse of electric current in order to generate an action which is similar to such pulsed laser. The invention described herein realizes such electro microsurgery in a physiological medium with a specific potential for applications in ophthalmology. Previous investigators who have considered pulsed electrical techniques and have seen cavitation bubble formation (R. Vorreuther, R. Corleis, T. Klotz, P. Bernards, U. Englemann, *J. Urology* **153:** 849-853 (1995) ; R. Lemery, T. K. Leung, E. Lavallee, A. Girard, M. Talajic, D. Roy, M. Montpetit, *Circulation* **83**(1): 279-293 (1991)) considered these bubbles either as an undesired side effect or as a means for shock wave generation for hard tissue destruction. These electrosurgical devices were designed for relatively high energy pulse generation: between 25 mJ and 40 J and with relatively long pulse duration: hundreds of microseconds. Such high energy pulses resulting in a few millimeter-sized cavitation bubbles cannot be applied to microsurgical applications such as those envisioned in delicate organs such as the eye. To accomplish such delicate cavitation bubble based microsurgery, a new electrical system based on an asymmetric microelectrode that enables generation of hundreds of thousands of cavitation bubbles, is described. This can become an alternative to endo-laser equipment in such areas as vitreoretinal surgery.

The concept can also be extended to the electroporation of individual cells and assemblies of cells in which the state of the prior art is a macroscopic device with macroscopic electrodes placed in a large bath with a solution of cells (M. Joersbo and J. Brunstedt, *Physiologia Plantarium* **81**: 256-264 (1991)). Instead of this a microelectrode for local electroporation of individual cells is used, or alternatively, an array of microelectrodes could be applied for poration of assemblies of cells.

In addition, by varying the nature of the characteristics of the electrical current the same device can be used for cavitation bubble cutting, electroporation or coagulation.

### State of Prior Art

Electrosurgical devices are widely used in surgery. The majority of these techniques are based on heating the tissue by an RF current and this local heat deposition causes one of the following processes: coagulation, and/or water evaporation. As a result, the only capability of such devices is to cut soft tissues by heat deposition which causes significant coagulation in the area surrounding the cut tissue. Such devices are totally useless for endolaser applications, for example in the eye. In addition to such RF techniques, DC pulsed electrical methodologies have not achieved a widespread acceptance because the absence of coagulation was considered an undesirable effect. In the past, cavitation bubbles were generated by DC pulsed methodologies. However, these techniques, which were designed for relatively high energy pulse generation (tens of milliJoules) with relatively long pulse durations (hundreds of microseconds), were only used as a means for shock wave generation for hard tissue destruction (R. Vorreuther, R. Corleis, T. Klotz, P. Bernards, U. Engelmann, *J*. *Urology* **153**: 849-853 (1995)). These high energy pulses resulted in cavitation bubbles with dimensions of a few millimeters, and these have no applicability to, for example, eye microsurgery in which considerably smaller bubbles are required. Laser-based techniques indicate that the pulse energies required to produce such bubbles are in the range of a few tens of microJoules and the pulse durations required are generally in a submicrosecond range (D. Palanker, I. Hemo, I. Turovets, H. Zauberman, A. Lewis, *Invest Ophthal. Vis. Sci.* **35**: 3835-3840 (1994) ; C. P. Lin, Y. K. Weaver, R. Birngruber, J. P. Fujimoto, C. A. Puliafito, *Lasers Surg. Med.* **15**: 44-53 (1994)). In addition to these differences in the time/energy characteristics of the available DC pulsed technologies as compared the present invention, it is required that any device for applications such as vitreoretinal surgery should be able to withstand tens of thousands of pulses. The previous high energy devices described above have a lifetime of less than 100 pulses (R. Vorreuther, R. Corleis, T. Klotz, P. Bernards, U. Engleman, *J. Urology* **153**: 849-853 (1995) and this is by far not sufficient for the microsurgical applications that are envisioned.

In terms of cell poration, the prior art were again macroscopic devices with macroscopic electrodes placed in a large bath (M. Joersbo and J. Brunstedt, *Physiologia Plantarium* **81**: 256-264 (1991)) with a solution of cells.

US-A-4 674 499 discloses a device with a microelectrode suitable for eye microsurgery. This disclosure does not mention a spark generator for generating extremely short electrical pulses producing very high voltages. Further there are two dimensions given therein for the electrode but below this there are also smaller dimensions referred to. There is no difference disclosed for the power supplied to these two differently dimensioned microelectrodes.

US-A-4 597 388 refers to an apparatus for removing an eye lens. There are no dimensions given therein for the electrodes and the power supply.

Both prior art documents disclose a polymeric insulation for the electrodes.

### Summary of the Invention

The device and method of the present invention as specified respectively in Claims 1 and 21 are based on producing submicrosecond high voltage discharges in physiological media with special protocols designed for the generation of cavitation bubbles for soft tissue microsurgery in a liquid environment as is possible today with certain pulsed lasers. The device consists of a combination of three elements:
- a specific microelectrode structure,
- specific electrical protocols to develop the required pulse characteristics to emulate pulsed laser microsurgery with cavitation bubble generation in the same device that can produce other electrical protocols for tissue coagulation and electroporation; and
- the above protocols determine the characteristics of the HV pulse generator that is to be used.

### Description of the Invention

The foregoing features of the invention are illustrated in the accompanying drawings, in which:
Figure 1A is a schematic illustration of a microelectrode in accordance with the invention, utilizing a single wire and having a tapered insulator with a flat tip;
Figure 1B is a schematic illustration of a microelectrode in accordance with the invention, utilizing a single wire and having a nontapered insulator with a flat tip;
Figure 1C is a modification of the device of Figure 1A, wherein the insulator has a rounded tip;
Figure 1D is a modification of the - device of Figure 1B, wherein the insulator has a rounded tip;
Figure 2 is a schematic diagram of a high voltage generator connected to the microelectrode of Figs. 1A-1D;
Figure 3A is a schematic illustration of a microelectrode in accordance with the invention, having multiple wires;
Figure 3B is a schematic illustration of a partially bent, single-wire microelectrode in accordance with the invention, having a flat tip;
Figure 3C is a schematic illustration of a single-wire side-firing microelectrode in accordance with the invention, having a flat tip;
Figure 4A and 4B are schematic top and side views, respectively, of a microelectrode array;
Figure 5A is a graphical illustration of the time dependence of electrode potential (U) and current passing through the exit surface of an electrode (-I) during a discharge, the electrode having a wire diameter of 25 *µ*m;
Figure 5B is a graphical illustration of a second discharge, following that illustrated in Fig. 5A;
Figure 6 is a sequence of micrographs illustrating the dynamics of cavitation bubbles generated at a potential (Uₘₐₓ) of 2.7kV, utilizing an electrode having a wire diameter of 20*µ*m and a tip diameter of 200 *µ*m, at a magnification of 100x, the delay between the electric pulse and the flash pulse being shown in *µ*s in each frame.

### The Microelectrodes

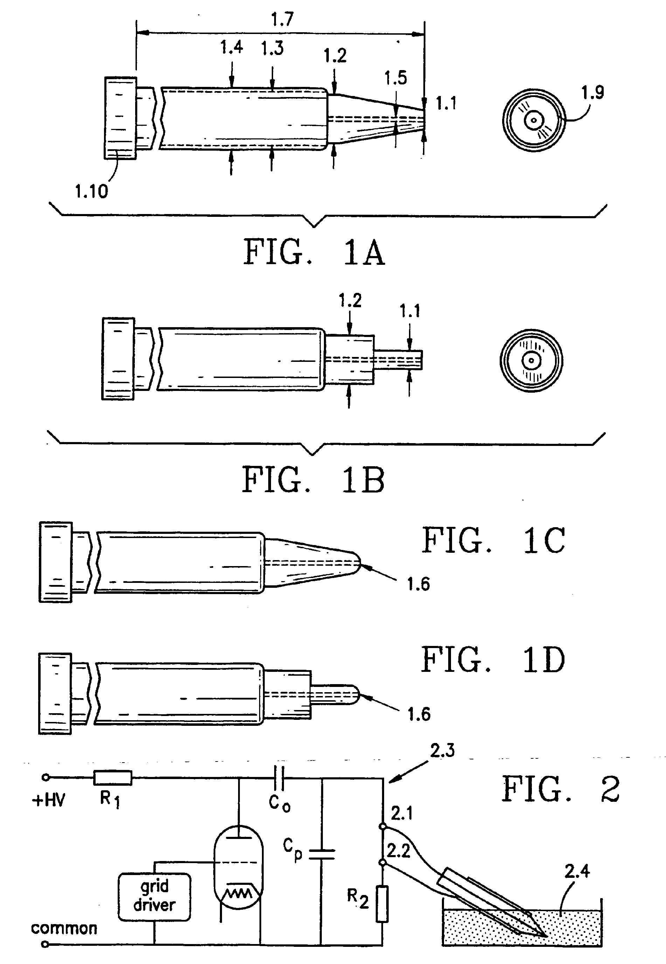

One way to form the microelectrodes, or needles, of the present invention is to seal a thin metal wire (1.5) into an insulator (1.2) with a variety of structures that can be tapered (Figures 1A & 1C) or have other geometries (Figures 1B & 1D). The wire (1.5) has a diameter at its exit from insulator (1.2) in the range of 1-100 microns. This insulator (1.2) and wire (1.5) creates an internal (main) electrode (1.1). A second electrode (1.3) is provided to mechanically protect the main electrode. This may be formed by coating the surrounding insulator (1.2) with a metallic coating (1.3) (see Figure 1A). The resulting external electrode can play the role of a protective metal cover for the main inner electrode (1.1). The geometry of this microelectrode and its connection to the output terminals (2.1) and (2.2) of the HV pulse generator (2.3) are schematically shown in Figure 2.

As shown in Fig. 2, the main electrode is placed in a conducting medium (2.4). The high voltage (HV) generator (2.3) produces electrical submicrosecond duration discharges through a gas layer in the conducting liquid-containing environment which produces cavitation bubbles with characteristics which emulate the action of pulsed lasers in a liquid medium. The bubbles are of sub-millimeter sizes. Such cavitation bubbles provide a mechanism for soft tissue microsurgery in a physiological medium, such as in the eye.

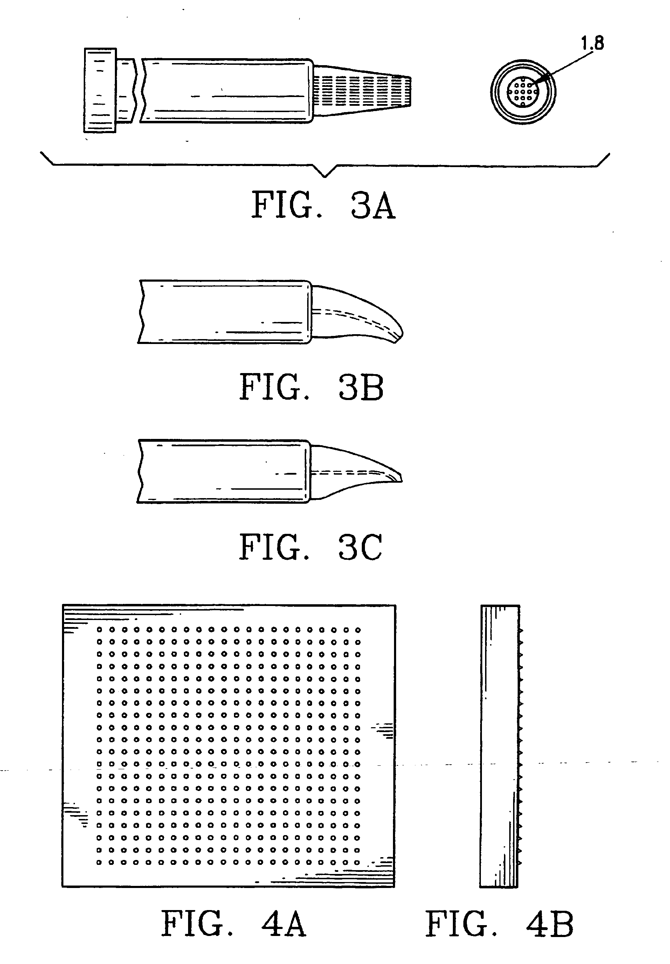

The microelectrode may be fabricated by pulling a glass microcapillary tube having a metal wire inside the tube. The tube is heated and pulled to produce the tapered shape of Fig. 1A, with the glass forming the insulator (1.2). Alternatively, the microelectrode can be fabricated using microlithography. The device may be used as a single device or multiple microelectrodes may be mounted in an array, as illustrated in Figs 4A and 4B, for use in microcutting or microperforation of materials, such as aggregates of cells.

The outer dimensions of the microelectrode (1.4) can be chosen according to the requirements of the application. For example, for microsurgical applications they could be similar to that of laser tips applied in endolaser microsurgery.

The diameter of the insulator around the exit of the main electrode (1.1) should be large enough to have mechanical strength while preventing the puncture of soft tissue. On the other hand, it should be small enough to enable it to reach the tissue to be cut at various angles. These requirements determine the range of the diameter of electrode (1.1) to be: 0.05 - 0.4mm. It is useful to have rounded edges of the insulator (Figure 1C, 1.6) in order to keep the electrode wire (1.5) in close proximity to the treated tissue when the tip is held at different angles relative to the tissue surface. The diameter of insulator (1.2) inside the second electrode (1.3) should be as large as possible to decrease the capacitance of the electrodes in the conductive liquid environment, and could be equal to the inner diameter of the external electrode (1.3). In certain cases (1.2) could be smaller than (1.3) in order to provide a gap (1.9) between electrode 1.3 and insulator (1.2) (Fig. 1A) to allow suction during the-treatment. Such suction allows lifting of treated tissues and the evacuation of gas bubbles and tissue debris that results from tissue cutting by the generated bubbles.

The outer diameter of the second (external) electrode (1.4) should be 0.9 - 1mm, as this is standard equipment for instruments used in certain microsurgical procedures used in vitreoretinal surgery.

The total length (1.7) of the microelectrode (Figure 1A) is 38 - 40mm to allow for access to all the areas inside the eye ball for such vitreoretinal surgery. Other microsurgical procedures may require other dimensions.

The same structure could be used with, for example, a set of multiple wires as the inner electrode (see Figure 3A, 1.8) or the tip can be partially bent in order to fire the cavitation bubble at an angle (Figure 3B) or can have a geometry that can produce a cavitation bubble at right angles to the axis of the electrode (Figure 3C). All the electrode configurations shown in Figs. 3A - 3C can also have electrode and insulator geometries with the structures that are seen in Figs. 1A - 1D.

### Electrical Protocols to Emulate Pulse Laser Induced Cavitation Bubbles

To achieve the high cutting efficiency of the pulsed laser treatments that are currently being developed, cavitation bubbles should be created fast enough for generation of high pressures and high boundary velocity and acceleration. These requirements determine the minimal peak power of the pulse. On the other hand, the cutting action should be local enough for prevention of extensive damage in the surroundings of a lesion. This requirement limits the total energy imposed on the bubble formation. Based on the experience of laser-induced cavitation (D. Palanker, I. Hemo, I. Turovets, H. Zauberman, A. Lewis, *Invest Ophthal. Vis. Sci* **35**: 3835-3840 (1994); C. P. Lin, Y. K. Weaver, R. Birngruber, J. G. Fujimoto, C. A. Puliafito, *Lasers Surg. Med.* **15**: 44-53 (1994)), the diameter of the cavitation bubble required for precise and effective cutting of vitreoretinal tissue should be in a range of 0.4 - 0.5 mm that corresponds to the bubble energies in a range of 3 to 6 *µ*J.

### The High Voltage Pulse Generator

The foregoing protocols determine the characteristics of the high voltage pulse generator. The electrode diameter 1.5 has to be capable of generating single pulses and pulse trains with a pulse duration varying in a range of 30 ns - 3 *µ*s, and the generator must be capable of varying the voltage amplitude in the range of 100 V - 10 kV. The peak current during the pulse can reach a few Amperes.

In addition to pulse generation aimed at cavitation bubble generation and tissue cutting, electrical pulses with lower voltage and, possibly, longer pulse duration could be applied with the above electrodes for electroporation of individual cells or for cell layers. Furthermore, the electrical power supply can also be amended so that this invention can also have the added capability of a coagulation.

### Extension of the Invention to Parallel Devices

Similar electrode geometries and pulse protocols can be envisioned to produce a parallel array of electrodes for the generation of a parallel array of cavitation bubbles and/or heating for producing bioelectromechanical field effects on multiple biological cells at one time (see Fig. 4).

### Applications of the Device

Numerous applications of the invented device are possible in the field of soft tissue microsurgery based on the cutting action of the generated cavitation bubbles. One of the most promising for the single electrode is vitreoretinal membrane removal, because the accepted mechanical peeling and cutting of such membranes is often associated with retinal damage. Furthermore, the present device can be used in all microsurgical procedures in physiological media (or other conducting liquids), including microsurgery of the internal organs.

In addition, with the modified electrical characteristics the device could also be used for bioelectromechanical effects of individual cells and cell layers, and coagulation of tissue.

Furthermore, the bent, cantilevered tips that produced in this invention can be of considerable value in other areas of science and technology. For example, such cantilevered electrodes can be produced such that the cantilever is very flexible (Force constants of a few N/m) and the tip is very small (0.05 µ). With such an extension of the present methodology, together with the pulsed protocols that have been developed, and that have been described herein, controlled alteration of surfaces can be effected that will allow fine lithographic patterning of such surfaces. **Experiments**

### Analysis of the Modes of the Electrical Discharge of the Device.

The pulse protocols that have been invented and are described herein were developed as a result of detailed experimentation that allowed close emulation of the effects of pulsed laser cavitation bubble generation.

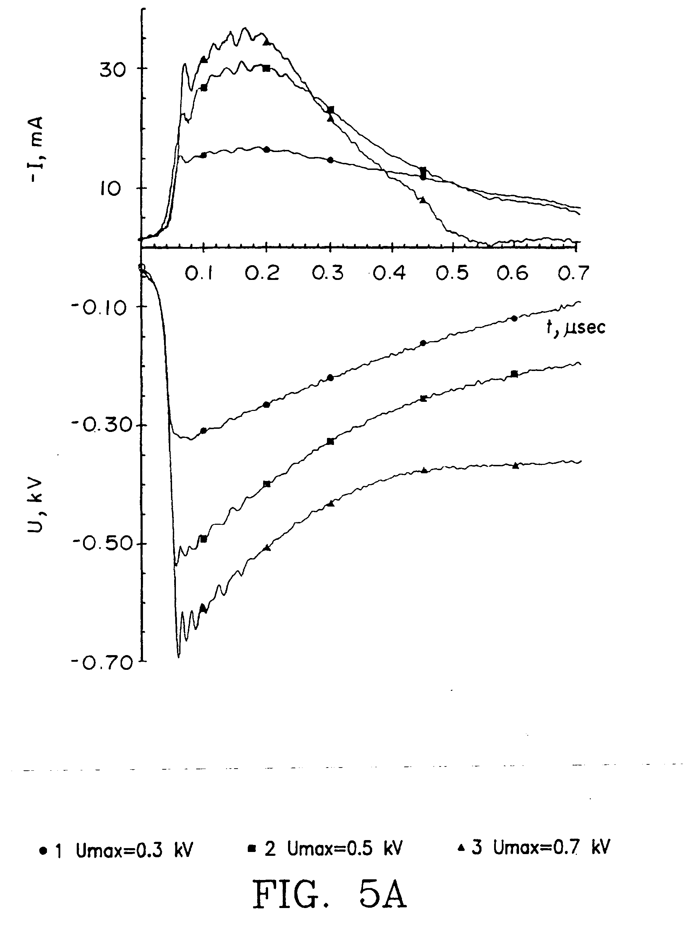

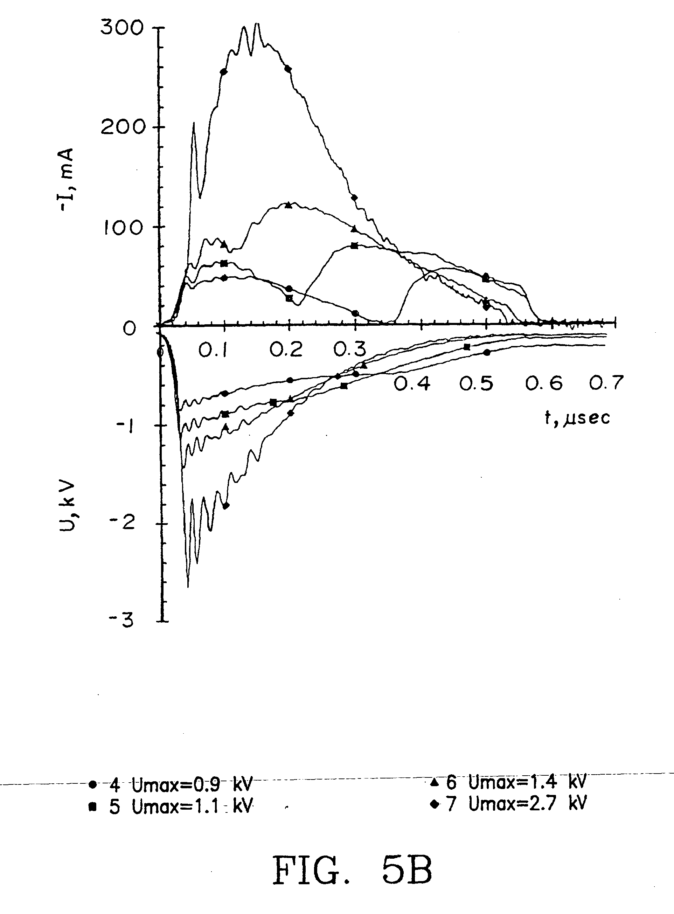

Specific examples of these experiments are described in this section. For example, for an electrode with a wire diameter (1.5) of 25 *µ*m the pulse profiles of the electrode potential (U) and the current passing through the exit surface of the electrode (I) are presented in Figure 5. At Uₘₐₓ =0.3 kV (Fig. 5A, curves 1) the voltage and current simultaneously decrease with a time constant of about 0.6 *µ*s. As the potential increases, the nature of the discharge changes: The current drops much faster after about 0.2 *µ*s, resulting in slowing down the voltage reduction (Fig. 5A, curves 2). At Uₘₐₓ = 0.7 kV (Fig. 5A, curves 3), the current falls to zero (switched off) when the potential is still at half of the maximum. This switching off of the current results from gas generation on the surface of the electrode that disconnects the liquid from the metal surface.

As the electric field in the gas layer becomes high enough, an electron avalanche is generated in this layer, that then propagates inside the liquid. This results in the second pulse of current generated after the first one (see Fig. 5B), and with increase of the voltage the delay between these two pulses decrease. At Uₘₐₓ higher than 1.4 kV (Figure 5B, curve 6), these two pulses completely overlap and at Uₘₐₓ 2.7 kV (Figure 5B, curve 7), they became indistinguishable. The discharge at the voltage range of 1.4 - 2.7 kV was accompanied by an emission of reddish light and a sound wave generation. At Uₘₐₓ = 2.6 kV, the dimensions of the lighted spot was about 7 µm. The best emulation of the laser cutting was achieved with a range of high voltage that is 2 - 2.7 kV.

### Cavitation Bubble Dynamics

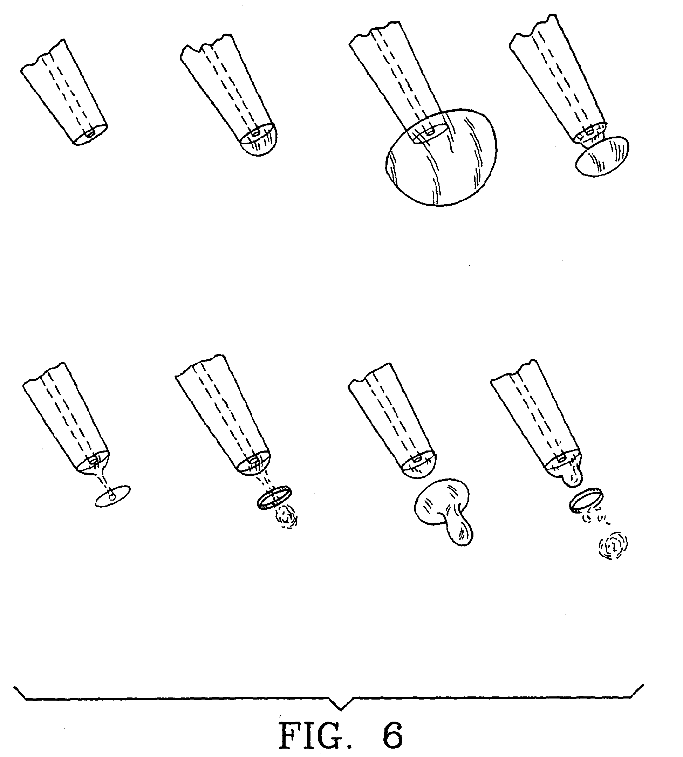

The sequence of micrographs of cavitation generated at the electrode with a 20 µm wire at Uₘₐₓ = 2.7 kV is shown in Fig. 6. The delay time between the electric pulse and the flash of the dye laser is shown (in *µ*s) in the corner of each frame. The spark generated in the vicinity of the electrode is clearly seen as a white spot in front of the wire. The average velocity of the bubble boundary during the first 1 *µ*s of the growth phase (frames 1,2) was about 90 m/s. The primary cavitation bubble grew in about 25 *µ*s (frames 2,3) reaching the maximal diameter of about 0.5 mm. During the collapse, the bubble had a mushroom-like shape (frame 4) that was eventually transformed to a ring and a stem connecting its center with the center of the tip (frame 5). The secondary bubbles were generated from both the ring and the stem (frames 6,7). These bubbles were ejected away from the tip at different velocities (about 5 and 17 m/s, respectively) and then collapsed and disappeared at distances of about 0.25 and 0.65 mm, respectively, at about 76 *µ*s after the pulse (frame 8).

### Tissue Cutting

Post-mortem fresh bovine eyes were prepared as an eyecup preparation: the anterior segments of the eyes and vitreous were removed and the eyecup was filled with Hartmann's physiological solution. For measurements of a cutting rate, 4-5 cuts of about 1 cm length were produced at the repetition rate of 30 Hz. Cutting efficiency was determined at the speed of the full depth cutting of retina. Cutting of retina with the rate exceeding 1 mm/s was observed at pulse energies of about 80 *µ*J/pulse with the electrode wire diameter varying in a range 10 - 25 *µ*m. The retinal tissue in the immediate vicinity of the ablated region looked normal, and the borders of the lesion were quite sharp and clean.

These experiments are the first step in demonstrating a variety of delicate surgical procedures that will evolve as a result of the application of this new device and method. For example, the microelectrode (1.1) can be connected to a catheter (1.10) or other support mechanism for manipulation. If desired, the gap 1.9 may serve as a conduit for the delivery of drugs to the regions of the tip of the microelectrode. Such delivery can be done simultaneously with the bubble cutting of the tissue.

## Claims

1. A device comprising:
a microelectrode incorporating a metal wire (1.5) inside an insulator (1.2) to form a concentric metal needle, said wire (1.5) having at its exit from said insulator an exit diameter in the range of 1 to 100 microns;
a high voltage pulse generator (2.3) connected to said wire (1.5) to generate in a liquid containing conductive medium (2.4) at said exit an electrical discharge having a duration in the range of 0.03 to 3 microseconds with a pulse energy in the range of 1-1000 *µ*J.

2. The device of claim 1, further including a second electrode (1.3) surrounding said insulator (1.2).

3. The device of claim 2, wherein said high voltage pulse generator (2.3) produces a voltage in the range of 100 V to 10 kV between said metal wire (1.5) and said second electrode (1.3).

4. The device of claim 1, wherein said insulator (1.2) is a glass microcapillary.

5. The device of claim 4, wherein said microcapillary is tapered, and further including a second electrode (1.3) surrounding said microcapillary.

6. The device of claim 5, wherein said second electrode (1.3) is a metal coating on said insulator (1.2).

7. The device of claim 5, wherein said second-electrode (1.3) has an outside diameter of no more than 1 mm.

8. The device of claim 7, wherein said outside diameter is between 0.9 mm and 1.0 mm.

9. The device of claim 5, wherein said conductive medium (2.4) is liquid, said electrical discharge producing in said conductive medium submillimeter-sized cavitation bubbles.

10. The device of claim 9, wherein said bubbles have diameters of between 0.4 and 0.5 mm.

11. The device of claim 1, wherein said high voltage pulse generator (2.3) produces pulses shaped to generate at said metal wire (1.5) exit a discharge that produces a cavitation bubble in said conductive medium (2.4) which will collapse after reaching a maximum diameter of 0.5 mm.

12. The device of claim 1, wherein said electrical discharge comprises submicrosecond sparks in said conductive medium (2.4) to produce cavitation bubbles having diameters of between 0.4 and 0.5 mm.

13. The device of claim 12, further including a second electrode (1.3) surrounding said insulator (1.2) and a gap (1.9) between said insulator (1.2) and said second electrode (1.3).

14. The device of claim 1, wherein said insulator (1.2) is pulled glass having a taper and an exit diameter of between 1 and 100 microns.

15. The device of claim 1, wherein said microelectrode is composed of multiple metal wires (1.8) inside said insulator (1.2).

16. The device of claim 1, wherein said microelectrode includes a tip bent at an angle with respect to the axis of the microelectrode.

17. The device of claim 16, wherein said microelectrode is mounted as a cantilever and is sufficiently flexible to detect surface variations of nanometer dimensions.

18. The device of claim 1, wherein said microelectrode has a flat tip, an angled tip, or a round tip (1.6).

19. The device of claim 1, further including multiple microelectrodes forming an array of parallel microelectrodes.

20. The device of at least one of the preceding claims*,*
wherein the microelectrode comprising a glass microcapillary (1.2) having a metal wire (1.5) inside the glass, the microcapillary being tapered to a point and coated with metal to form a concentric metal needle, said metal wire (1.5) having an exit diameter in the range of 1-100 microns; and
the high voltage pulse generator (2.3) connected to said wire (1.5) to generate a submicrosecond duration discharge in a conducting liquid containing medium (2.4) with pulse energies in the range of 1-1000 µJ to produce a cavitation bubble, said discharge having a duration sufficiently short to produce a bubble having a diameter no greater than 0.5 mm in said conducting medium (2.4) with characteristics that emulate the action of pulsed lasers in said conducting medium.

21. A method of cutting of in-vitro cell aggregations comprising:
providing a microelectrode incorporating a metal wire inside on insulator to form a concentric metal needle, said wire having at its exit from said insulator an exit diameter in the range of 1 to 100 microns ;
providing a high voltage generator connected to said wire to generate a high-voltage, submicrosecond duration electrical discharge in a conducting liquid-containing medium (2.4) with pulse energies in the range of 1-1000 µJ to produce a cavitation bubble in said medium;
causing said discharge to have a duration in the range of 0.030 to 3.0 microseconds which is sufficiently short to produce a bubble having a diameter no greater than about 0.5 mm in said medium with characteristics that emulate the action of pulsed lasers in said medium; and
directing said cavitation bubble towards said cell aggregations.

22. The method of claim 21, further including generating said cavitation bubbles so as to cause the bubbles to collapse after reaching a maximum diameter of 0.5 mm.

23. The method of claim 21, including generating a parallel array of said cavitation bubbles in said medium.

24. The method of claim 21, further including generating said cavitation bubbles so as to cause said bubbles to collapse after reaching a maximum diameter of between about 0.4 and 0.5 mm.

25. The method of claim 21, wherein generating a high-voltage discharge includes:
forming a microelectrode having a single wire (1.5) inside an insulator (1.2) and a second electrode (1.3) surrounding the insulator (1.2); and
applying a voltage pulse having an amplitude in the range of 100 V to 10 kV and having a duration in the range of 0.030 and 3.0 microseconds between said wire (1.5) and said second electrode (1.3) to generate said discharge.

26. The method of claim 25, where said discharge causes said cavitation bubble to collapse after reaching a maximum diameter of between about 0.4 and 0.5 mm.

27. The method of claim 21, further including:
providing said microelectrode with an electrical conductor (1.3) surrounding the insulator (1.2), and
wherein generating said electrical discharge includes generating a pulse having a duration in the said range of 0.03 to 3.0 microseconds to produce an electron avalanch having a pulse energy sufficient to produce said cavitation bubble.

28. The method of claim 27, wherein generating said pulse includes applying a voltage between said metal wire (1.5) and said electrical conductor (1.3).

29. The method of claim 28, wherein generating said pulse includes producing an electron avalanch having a pulse energy in the range of 1-1000 µJ.

## Patentansprüche

1. Vorrichtung, welche umfasst:
eine Mikroelektrode, die einen Metalldraht (1.5) im Inneren eines Isolators (1.2) aufnimmt, um eine konzentrische Metallnadel zu bilden, wobei der Draht (1.5) an seinem Austritt aus dem Isolator einen Austrittsdurchmesser im Bereich von 1 bis 100 µm aufweist;
einen Hochspannungsimpulsgenerator (2.3), der mit dem Draht (1.5) verbunden ist, um in einem eine Flüssigkeit enthaltenden leitfähigen Medium (2.4) am Austritt eine elektrische Entladung mit einer Dauer im Bereich von 0,03 bis 3 Mikrosekunden mit einer Impulsenergie im Bereich von 1-1000 µJ zu erzeugen.

2. Vorrichtung nach Anspruch 1, die ferner eine zweite Elektrode (1.3) beinhaltet, die den Isolator (1.2) umgibt.

3. Vorrichtung nach Anspruch 2, bei dem der Hochspannungsimpulsgenerator (2.3) eine Spannung im Bereich von 100 V bis 10 kV zwischen dem Metalldraht (1.5) und der zweiten Elektrode (1.3) erzeugt.

4. Vorrichtung nach Anspruch 1, bei der der Isolator (1.2) eine Glas-Mikrokapillare ist.

5. Vorrichtung nach Anspruch 4, bei der die Mikrokapillare verjüngt ist und die ferner eine zweite Elektrode (1.3) umfasst, die die Mirkokapillare umgibt.

6. Vorrichtung nach Anspruch 5, bei der die zweite Elektrode (1.3) eine Metallbeschichtung auf dem Isolator (1.2) ist.

7. Vorrichtung nach Anspruch 5, bei der die zweite Elektrode (1.3) einen Außendurchmesser von nicht mehr als 1 mm aufweist.

8. Vorrichtung nach Anspruch 7, bei der der Außendurchmesser zwischen 0,9 mm und 1,0 mm liegt.

9. Vorrichtung nach Anspruch 5, bei der das leitfähige Medium (2.4) eine Flüssigkeit ist, wobei die elektrische Entladung ein dem leitfähigen Medium Blasen mit einem Hohlraum mit einer Größe unterhalb eines Millimeters erzeugt.

10. Vorrichtung nach Anspruch 9, bei der die Blasen Durchmesser zwischen 0,4 und 0,5 mm aufweisen.

11. Vorrichtung nach Anspruch 1, bei der der Hochspannungsimpulsgenerator (2.3) Impulse erzeugt, die so geformt sind, dass sie am Austritt des Metalldrahts (1.5) eine Entladung erzeugen, die eine Blase mit Hohlraum in dem leitfähigen Medium (2.4) erzeugt, die nach Erreichen eines maximalen Durchmessers von 0,5 mm kollabiert.

12. Vorrichtung nach Anspruch 1, bei der die elektrische Entladung Funken unterhalb einer Mikrosekunde in dem leitfähigen Medium (2.4) umfasst, um Blasen mit Hohlräumen mit Durchmessern zwischen 0,4 und 0,5 mm zu erzeugen.

13. Vorrichtung nach Anspruch 12, die ferner eine zweite Elektrode (1.3), die den Isolator (1.2) umgibt, und einen Spalt (1.9) zwischen dem Isolator (1.2) und der zweiten Elektrode (1.3) umfasst.

14. Vorrichtung nach Anspruch 1, bei der der Isolator (1.2) gezogenes Glas mit einer Verjüngung und einem Austrittsdurchmesser zwischen 1 und 100 µm ist.

15. Vorrichtung nach Anspruch 1, bei der die Mikroelektrode aus mehreren Metalldrähten (1.8) im Inneren des Isolators (1.2) besteht.

16. Vorrichtung nach Anspruch 1, bei der die Mikroelektrode eine Spitze beinhaltet, die bezüglich der Achse der Mikroelektrode um einen Winkel gebogen ist.

17. Vorrichtung nach Anspruch 16, bei der die Mikroelektrode als Ausleger angebracht und ausreichend flexible ist, um Oberflächenvariationen mit Nanometer-Abmessungen zu erfassen.

18. Vorrichtung nach Anspruch 1, bei der die Mikroelektrode eine flache Spitze, eine gewinkelte Spitze oder eine runde Spitze (1.6) aufweist.

19. Vorrichtung nach Anspruch 1, die ferner mehrere Mikroelektroden beinhaltet, die eine Anordnung paralleler Mikroelektroden bilden.

20. Vorrichtung nach wenigstens einem der vorhergehenden Ansprüche, bei der die Mikroelektrode eine Glas-Mikrokapillare (1.2) mit einem Metalldraht (1.5) im Glas umfasst, wobei die Mikrokapillare zu einer Spitze verjüngt und mit Metall beschichtet ist, um eine konzentrische Metallnadel zu bilden, wobei der Metalldraht (1.5) einen Austrittsdurchmesser im Bereich von 1-100 µm aufweist; und
der Hochspannungspulsgenerator (2.3) mit dem Draht (1.5) verbunden ist, um eine Entladung mit einer Dauer unterhalb einer Mikrosekunde im leitfähigen, eine Flüssigkeit enthaltenden Medium (2.4) mit Impulsenergien im Bereich von 1-1000 µJ zu erzeugen, um eine Blase mit Hohlraum zu erzeugen, wobei die Entladung eine Dauer hat, die ausreichend kurz ist, um eine Blase mit einem Durchmesser nicht größer als 0,5 mm in dem leitfähigen Medium (2.4) mit Eigenschaften zu erzeugen, die die Wirkung von gepulsten Ladern in dem leitfähigen Medium nachahmen.

21. Verfahren zum Schneiden von In-Vitro-Zellanhäufungen, welches umfasst:
Bereitstellen einer Mikroelektrode, die einen Metalldraht im Inneren eines Isolators aufnimmt, um eine konzentrische Metallnadel zu bilden, wobei der Draht an seinem Austritt aus dem Isolator einen Austrittsdurchmesser im Bereich von 1 bis 100 µm aufweist;
Bereitstellen eines Hochspannungsgenerators, der mit dem Draht verbunden ist, um eine elektrische Entladung mit hoher Spannung und einer Dauer unterhalb einer Mikrosekunde in einem leitfähigen, eine Flüssigkeit enthaltenden Medium (2.4) mit Impulsenergien im Bereich von 1-1000 µJ zu erzeugen, um in dem Medium eine Blase mit Hohlraum zu erzeugen;
Bewirken, dass die Entladung eine Dauer im Bereich von 0,030 bis 3,0 Mikrosekunden hat, die ausreichend kurz ist, um eine Blase mit einem Durchmesser von nicht mehr als etwa 0,5 mm in dem Medium mit Eigenschaften zu erzeugen, die die Wirkung eines gepulsten Lasers in dem Medium nachahmen; und
Richten der Blasen mit Hohlraum auf die Zellanhäufungen.

22. Verfahren nach Anspruch 21, das ferner das Erzeugen der Blasen mit Hohlraum beihaltet, um zu bewirken, dass die Blasen nach dem Erreichen eines maximalen Durchmessers von 0,5 mm kollabieren.

23. Verfahren nach Anspruch 21, das das Erzeugen einer parallelen Anordnung der Blasen mit Hohlraum im Medium beinhaltet.

24. Verfahren nach Anspruch 21, das ferner das Erzeugen der Blasen mit Hohlraum beinhaltet, um zu bewirken, dass die Blasen nach dem Erreichen eines maximalen Durchmessers zwischen etwa 0,4 und 05, mm kollabieren.

25. Verfahren nach Anspruch 21, bei dem das Erzeugen einer Hochspannungsentladung beinhaltet:
Ausbilden einer Mikroelektrode mit einem einzigen Draht (1.5) im Inneren eines Isolators (1.2) und einer zweiten Elektrode (1.3) die den Isolator (1.2) umgibt; und
Anlegen eines Spannungsimpulses mit einer Amplitude im Bereich von 100 V bis 10 kV und mit einer Dauer im Bereich von 0,030 bis 3,0 Mikrosekunden zwischen dem Draht (1.5) und der zweiten Elektrode (1.3), um die Entladung zu erzeugen.

26. Verfahren nach Anspruch 25, bei dem die Entladung bewirkt, dass die Blase mit Hohlraum nach Erreichen eines maximalen Durchmessers zwischen etwa 0,4 und 0,5 mm kollabiert.

27. Verfahren nach Anspruch 21, das ferner beinhaltet:
Bereitstellen der Mikroelektrode mit einem elektrischen Leiter (1.3), der den Isolator (1.2) umgibt; und
bei dem das Erzeugen der elektrischen Entladung das Erzeugen eines Impulses mit einer Dauer im Bereich von 0,03 bis 3,0 Mikrosekunden beinhaltet, um eine Elektronenlawine mit einer Impulsenergie zu erzeugen, die ausreicht, um die Blase mit Hohlraum zu erzeugen.

28. Verfahren nach Anspruch 27, bei dem das Erzeugen des Impulses das Anlegen einer Spannung zwischen dem Metalldraht (1.5) und dem elektrischen Leiter (1.3) beinhaltet.

29. Verfahren nach Anspruch 28, bei dem das Erzeugen des Impulses das Erzeugen einer Elektronenlawine mit einer Impulsenergie im Bereich von 1-1000 µJ beinhaltet.

## Revendications

1. Dispositif comprenant :
une microélectrode incorporant un câble métallique (1.5) à l'intérieur d'un isolant (1.2) pour former une aiguille métallique concentrique, ledit câble (1.5) ayant, au niveau de sa sortie dudit isolant, un diamètre de sortie dans la plage de 1 à 100 microns ;
un générateur d'impulsions à haute tension (2.3) connecté audit câble (1.5) pour générer dans un milieu conducteur contenant un liquide (2.4) au niveau de ladite sortie une décharge électrique ayant une durée dans la plage de 0,03 à 3 microsecondes avec une énergie d'impulsion dans la plage de 1 à 1 000 µJ.

2. Dispositif selon la revendication 1, incluant en outre une deuxième électrode (1.3) entourant ledit isolant (1.2).

3. Dispositif selon la revendication 2, dans lequel ledit générateur d'impulsions à haute tension (2.3) produit une tension dans la plage de 100 V à 10 kV entre ledit câble métallique (1.5) et ladite deuxième électrode (1.3).

4. Dispositif selon la revendication 1, dans lequel ledit isolant (1.2) est un microcapillaire en verre.

5. Dispositif selon la revendication 4, dans lequel ledit microcapillaire est conique, et incluant en outre une deuxième électrode (1.3) entourant ledit microcapillaire.

6. Dispositif selon la revendication 5, dans lequel ladite deuxième électrode (1.3) est un revêtement métallique sur ledit isolant (1.2).

7. Dispositif selon la revendication 5, dans lequel ladite deuxième électrode (1.3) a un diamètre externe inférieur à 1 mm.

8. Dispositif selon la revendication 7, dans lequel ledit diamètre extérieur est compris entre 0,9 mm et 1,0 mm.

9. Dispositif selon la revendication 5, dans lequel ledit milieu conducteur (2.4) est liquide, ladite décharge électrique produisant dans ledit milieu conducteur des bulles de cavitation de l'ordre des inframillimètres.

10. Dispositif selon la revendication 9, dans lequel lesdites bulles ont des diamètres compris entre 0,4 et 0,5 mm.

11. Dispositif selon la revendication 1, dans lequel ledit générateur d'impulsions à haute tension (2.3) produit des impulsions formées pour générer, au niveau de la sortie dudit câble métallique (1.5), une décharge qui produit une bulle de cavitation dans ledit milieu conducteur (2.4) qui se rompt après avoir atteint un diamètre maximum de 0,5 mm.

12. Dispositif selon la revendication 1, dans lequel ladite décharge électrique comprend des étincelles de l'ordre des inframicrosecondes dans ledit milieu conducteur (2.4) pour produire des bulles de cavitation ayant des diamètres compris entre 0,4 et 0,5 mm.

13. Dispositif selon la revendication 12, incluant en outre une deuxième électrode (1.3) entourant ledit isolant (1.2) et un intervalle (1.9) entre ledit isolant (1.2) et ladite deuxième électrode (1.3).

14. Dispositif selon la revendication 1, dans lequel ledit isolant (1.2) est du verre étiré ayant un cône et un diamètre de sortie compris entre 1 et 100 microns.

15. Dispositif selon la revendication 1, dans lequel ladite microélectrode est composée de plusieurs câbles métalliques (1.8) à l'intérieur dudit isolant (1.2).

16. Dispositif selon la revendication 1, dans lequel ladite microélectrode inclut une pointe inclinée selon un angle par rapport à l'axe de la microélectrode.

17. Dispositif selon la revendication 16, dans lequel ladite microélectrode est montée en porte à faux et est suffisamment flexible pour détecter des variations en surface des dimensions de l'ordre des nanomètres.

18. Dispositif selon la revendication 1, dans lequel ladite microélectrode a une pointe plate, une pointe an biais, ou une pointe arrondie (1.6).

19. Dispositif selon la revendication 1, incluant en outre plusieurs microélectrodes formant un groupe de microélectrodes parallèles.

20. Dispositif selon au moins l'une des revendications précédentes, dans lequel la microélectrode comprend un microcapillaire en verre (1.2) ayant un câble métallique (1.5) à l'intérieur du verre, le microcapillaire étant conique en un point et revêtu de métal pour former une aiguille métallique concentrique, ledit câble métallique (1.5) ayant un diamètre de sortie dans la plage de 1 à 100 microns ; et le générateur d'impulsions à haute tension (2.3) est connecté audit câble (1.5) pour générer une décharge d'une durée de l'ordre des inframicrosecondes dans un milieu contenant un liquide conducteur (2.4) grâce à des énergies d'impulsions dans la plage de 1 à 1 000 µJ pour produire une bulle de cavitation, ladite décharge ayant une durée suffisamment courte pour produire une bulle ayant un diamètre inférieur à 0,5 mm dans ledit milieu conducteur (2.4) avec des caractéristiques qui imitent l'action des lasers à impulsion dans ledit milieu conducteur.

21. Procédé de découpe de regroupements de cellules in vitro, comprenant les étapes consistant à :
fournir une microélectrode incorporant un câble métallique à l'intérieur d'un isolant pour former une aiguille métallique concentrique, ledit câble ayant, au niveau de sa sortie dudit isolant, un diamètre de sortie dans la plage de 1 à 100 microns ;
fournir un générateur à haute tension connecté audit câble pour générer une décharge électrique dont la durée se compte en inframicrosecondes, à haute tension, dans un milieu contenant un liquide conducteur (2.4) avec des énergies d'impulsions dans la plage de 1 à 1 000 µJ pour produire une bulle de cavitation dans ledit milieu ;
pousser ladite décharge à avoir une durée dans la plage de 0,030 à 3,0 microsecondes, ce qui est suffisamment court pour produire une bulle ayant un diamètre inférieur à environ 0,5 mm dans ledit milieu, avec des caractéristiques qui émulent l'action des lasers à impulsion dans ledit milieu ; et
diriger lesdites bulles de cavitation vers lesdits regroupements de cellules.

22. Procédé selon la revendication 21, incluant en outre la génération desdites bulles de cavitation de manière à pousser les bulles à se rompre après avoir atteint un diamètre maximum de 0,5 mm.

23. Procédé selon la revendication 21, incluant la génération d'un groupe parallèle desdites bulles de cavitation dans ledit milieu.

24. Procédé selon la revendication 21, incluant en outre la génération desdites bulles de cavitation de manière à pousser lesdites bulles à se rompre après avoir atteint un diamètre maximum compris entre environ 0,4 et 0,5 mm.

25. Procédé selon la revendication 21, dans lequel la génération d'une décharge à haute tension inclut :
la formation d'une microélectrode ayant un câble unique (1.5) à l'intérieur d'un isolant (1.2) et une deuxième électrode (1.3) entourant l'isolant (1.2) ; et
l'application d'une impulsion de tension ayant une amplitude dans la plage de 100 V à 10 kV et ayant une durée dans la plage de 0,030 et 3,0 microsecondes entre ledit câble (1.5) et ladite deuxième électrode (1.3) pour générer ladite décharge.

26. Procédé selon la revendication 25, dans lequel ladite décharge pousse ladite bulle de cavitation à se rompre après avoir atteint un diamètre maximum compris entre environ 0,4 et 0,5 mm.

27. Procédé selon la revendication 21, incluant en outre les étapes consistant à :
doter ladite microélectrode (1.5) d'un conducteur électrique (1.3) entourant l'isolant (1.2), et
dans lequel la génération de ladite décharge électrique inclut la génération d'une impulsion ayant une durée dans ladite plage de 0,03 à 3,0 microsecondes pour produire une avalanche d'électrons ayant une énergie d'impulsion suffisante pour produire ladite bulle de cavitation.

28. Procédé selon la revendication 27, dans lequel la génération de ladite impulsion inclut l'application d'une tension entre ledit câble métallique (1.5) et ledit conducteur électrique (1.3).

29. Procédé selon la revendication 28, dans lequel la génération de ladite impulsion inclut la production d'une avalanche d'électrons ayant une énergie d'impulsion dans la plage de 1 à 1 000 µJ.
